Europäisches Patentamt

European Patent Office

Office européen des brevets

(19)

(11) Numéro de publication: **0 148 661 B1**

(12) **FASCICULE DE BREVET EUROPEEN**

(45) Date de publication du fascicule du brevet:
15.07.87

(21) Numéro de dépôt: **84402406.7**

(22) Date de dépôt: **26.11.84**

(51) Int. Cl.⁴: **A 61 F 2/22**

(54) **Pompe sanguine implantable.**

(30) Priorité: **28.12.83 FR 8320933**

(43) Date de publication de la demande:
**17.07.85 Bulletin 85/29**

(45) Mention de la délivrance du brevet:
**15.07.87 Bulletin 87/29**

(84) Etats contractants désignés:
**AT BE CH DE FR GB IT LI LU NL SE**

(56) Documents cités:
**GB - A - 2 054 062**
**GB - A - 2 102 504**
**US - A - 3 328 255**
**US - A - 3 513 486**
**US - A - 4 008 710**

(73) Titulaire: **CLINIQUE DE LA RESIDENCE DU PARC, Rue Gaston Berger, F-13362 Marseille Cedex 10 (FR)**
Titulaire: **Pol, Vincent, 8, boulevard Rivet, F-13008 Marseille (FR)**
Titulaire: **Dumas, Jean-Claude, Villa La Bohême Quartier les Ratons, F-13390 Auriol (FR)**

(72) Inventeur: **Pol, Vincent, 8, Boulevard Rivet, F-13008 Marseille (FR)**
Inventeur: **Dumas, Jean-Claude, Villa La Bohême Quartier Les Ratons, F-13390 Auriol (FR)**

(74) Mandataire: **Martin, Jean-Jacques et al, Cabinet REGIMBEAU 26, Avenue Kléber, F-75116 Paris (FR)**

ACTORUM AG

## Description

La présente invention concerne une pompe sanguine implantable.

Cette pompe sanguine sera principalement décrite comme une assistance ventriculaire, par exemple destinée à remédier temporairement ou définitivement aux défaillances du ventricule gauche. Mais les enseignements de l'invention peuvent également être appliqués à la réalisation d'une prothèse cardiaque totale, en prévoyant deux ventricules associés pourvus chacun de leurs valvules propres.

De nombreux dispositifs ont été proposés pour réaliser une prothèse vertriculaire cardiaque. Il s'agit le plus souvent de pompes de type péristaltique, c'est-à-dire comprenant une poche déformable reliée à la circulation sanguine par deux valves d'entrée et de sortie. Cette poche est comprimée en lui appliquant une contrainte extérieure, en général produite par un dispositif pneumatique actionné, de façon contrôlée, par une source de pression (voir par exemple US-A-4 008 710).

Ce type de pompe a, du point de vue physiologique, un comportement proche de celui de la nature (poche contractile), ce qui procure l'avantage essentiel de rendre pratiquement inexistant le phénomène d'hémolyse (destruction des globules rouges), en réduisant considérablement les phénomènes de chocs, turbulences, brusques variations de pression, ... dans le fluide sanguin. La poche est en effet déformée par une contrainte extérieure; elle ne contient donc aucun organe mobile, tel qu'un piston, en contact direct avec le sang.

Un autre avantage des pompes péristaltiques est de fonctionner toujours avec une admission sanguine sous pression nulle ou faiblement négative. On évite ainsi les risques de collapsus en cas de retor veineux insuffisant. Ces risques, susceptibles d'entraîner un dysfonctionnement de la pompe et une mort rapide du sujet par écrasement de la cavité auriculaire et/ou des vaisseaux, sont inhérents aux pompes à fonctionnement volumétrique, qui nécessitent soit un asservissement très poussé du débit à la pression sanguine, soit la présence d'un volume tampon qui ne peut être envisagé que pour une circulation extracorporelle, en raison de l'encombrement supplémentaire résultant.

Dans les pompes proposées, l'entraînement pneumatique est cependant un obstacle à la réalisation d'une prothèse entièrement implantable et autonome: en effet, le dispositif pneumatique est tout d'abord volumineux (sources de pression, électrovannes et dispositif de régulation pneumatique), ce qui nécessite un appareillage extérieur au patient et relié à celui-ci par des liaisons pneumatiques.

Ensuite et surtout, le rendement global d'un compresseur pneumatique est mauvais, ce qui nécessite des sources d'énergie électrique de grande capacité. Ces souces sont lourdes et volumineuses et surtout, du fait du faible rendement de la conversion électropneumatique, dégagent des quantités de chaleur importantes incompatibles avec la thermorégulation du corps humain.

Enfin, un coeur à entraînement pneumatique utilise des diaphragmes minces (pour la transformation de l'énergie pneumatique fournie en énergie mécanique de pompage) qui sont particulièrement exposés à un risque de rupture, notamment à la suite de calcifications ponctuelles qui fragilisent leur paroi.

Le document US-A-3 513 486 propose une pompe cardiaque implantable comportant une poche déformable logée dans une coque extérieure, ladite poche étant comprimée alternativement par un dispositif mécanique entraîné par un moteur électrique. Ce moteur électrique étant disposé à l'extérieur du patient, une implantation entièrement intracorporelle n'est pas réalisable avec ce type de pompe.

L'invention a pour objet de proposer une nouvelle pompe cardiaque qui remédie à ces inconvénients.

La pompe selon l'invention est du type péristaltique, ce qui lui permet de bénéficier des avantages liés à l'absence d'organe mobile à l'intérieur de la poche (d'où très faible hémolyse), et au fonctionnement non volumétrique (d'où suppression du risque de collapsus veineux).

L'un des buts de la présente invention est de réaliser une prothèse, ventriculaire ou cardiaque, entièrement inplantable et autonome. En effet, grâce à un excellent rendement d'ensemble de la conversion d'énergie électrique en énergie mécanique de pompage, il est possible de limiter la consommation à des valeurs compatibles avec des sources électriques de faibles dimensions (batteries ou générateur isotopique) qui pourraient être implantées ou tout au moins emportées de façon commode par le patient de manière à rendre ce dernier autonome dans ses déplacements.

L'excellent rendement permet également de limiter les dissipations thermiques à des valeurs compatibles avec la thermorégulation naturelle. On verra en effet que la puissance totale consommée par la pompe (puissance mécanique développée + pertes thermiques) est du même ordre de grandeur que la puissance physiologique théorique d'un coeur naturel.

En outre, la disposition des différents éléments constituant la pompe de l'invention permet, sans réduction des performances, de réaliser celle-ci avec une forme d'ensemble et de dimensions voisines de celles d'un organe naturel. Cette compacité facilite l'implantation, unique ou en double, de l'organe artificiel.

On verra également que celui-ci présente une certaine souplesse lui permettant d'absorber les mouvements du thorax (respiration, toux), de la même façon qu'un coeur naturel. On évite ainsi un cetain nombre de difficultés rencontrées lors de l'implantation d'un organe rigide, ce qui était le cas de certaines réalisations proposées jusqu'à présent.

Enfin, on verra également que, outre une possibilité d'asservissement programmé du régime de la pompe (débit, fréquence, forme d'onde) à la pression sanguine, la pompe réalise déjà, de par sa structure propre, une auto-adaptation: en effet, si la pression sanguine vient à augmenter, le volume du ventricule tendra à augmenter par déformation élastique. Dans ces conditions, et sans changement de la fréquence cardiaque ni de la forme de l'onde de pression, le volume éjecté à chaque pulsation va augmenter, améliorant ainsi l'irrigation du patient. Dans ce cas, la prothèse devient plus performante que l'organe naturel, dont les tissus ne permettent pas cette dilatation ventriculaire sous l'effet d'une augmentation de pression sanguine.

Pour atteindre ces différents buts, selon l'invention, la pompe comprend:

une coque extérieure, avantageusement déformable dans son ensemble,

au moins une poche fermée élastiquement déformable, alimentée en fluide sanguin par une valve d'entrée, et susceptible de refouler ce fluide par une valve de sortie,

et est caractérisée en ce que ladite poche a une forme libre sensiblement plate et est placée contre la paroi intérieure de la coque de façon à présenter deux parois qui, ainsi courbées tournent toutes deux leur concavité vers l'intérieur de la coque, ces deux parois s'étendant entre deux régions allongées longitudinales, formant extrémités proximale et distale,

et en ce qu'elle comprend en outre un tambour, à la périphérie duquel est reliée l'extrémité proximale de la poche,

un étrier, monté à rotation sur l'axe longitudinal du tambour,

des moyens pour réaliser une liaison de traction entre l'étrier et l'extrémité distale de la poche,

des moyens pour entraîner en rotation relative et antagoniste, sur une fraction de tour, l'étrier et le tambour, dans un sens permettant à ce dernier de venir en contact de roulement avec la paroi de la poche, tandis que l'étrier maintient l'extrémité distale et tend à la rapprocher du tambour.

De cette façon, on réalise la compression de la poche sous l'effet d'un enroulement de celle-ci sur le tambour, tout en faisant en sorte que les positions successives de l'ensemble tambour-étrier suivent un mouvement de moindre résistance entre la réaction de compression de la poche et la réaction de traction de l'extrémité distale de la poche.

Le choix d'une poche fermée déformable comme volume de pompage permet de conserver les avantages procurés par le fonctionnement péristaltique, exposés plus haut. Cette poche a d'ailleurs une structure assimilable à celle d'une cavité physiologique virtuelle (ou, plus précisément, semivirtuelle): une telle cavité est une cavité qui, lorsqu'elle est vide, occupe un volume pratiquement nul, ses parois intérieures venant alors en contact. De la même façon, la poche utilisée dans l'invention, du fait de sa forme libre plate, forme une cavité qui n'existe quasiment pas au repos (c'est-à-dire en l'absence de pression sanguine). En fait, on préfère laisser subsister une lame liquide de très faible épaisseur (1 à 1,5 mm) pour limiter le risque d'apparition de phénomènes d'hémolyse; c'est pour cela que cette poche définit une cavité «quasi-virtuelle».

En ce qui concerne la coque, celle-ci a pour première fonction de constituer une surface d'appui, pendant au moins une partie de la course de la pompe; ceci notamment au démarrage, où la poche remplie de sang a tendance à être repoussée par le tambour qui vient la comprimer. Le maintien de l'appui contre la coque après le démarrage peut permettre en outre une excellente évacuation de la poche, celle-ci étant déformée entre le tambour et la coque.

De préférence, il est prévu une liaison entre l'étrier et la coque extérieure, cette liaison étant une liaison lâche permettant un pivotement de l'étrier par rapport à la coque, au moins autour d'un axe longitudinal.

De préférence, le matériau constituant la coque est un matériau possédant une mémoire géométrique propre. Ceci améliore la restitution d'énergie: la mémoire propre du matériau s'ajoute en effet à la déformation résultant du déplacement relatif du tambour et de l'étrier.

Ceci permet à la coque de réaliser une seconde fonction d'accumulateur d'énergie élastique, ce qui améliore le fonctionnement de la pompe à la fois de façon qualitative (forme de l'onde de pression) et de façon quantitative (rendement d'ensemble, pression d'éjection). L'énerige élastique de déformation de la poche est en effet restituée au système au moment du démarrage, c'est-à-dire au moment où cette énergie est la plus utile pour assister le moteur, celui-ci étant en court-circuit en début de compression de la poche. Cet effet de restitution de l'énergie élastique de la coque est d'ailleurs comparable à la restitution d'énergie des fibres cardiaques, dans un coeur naturel.

Avantageusement, les moyens pour réaliser une liaison de traction entre l'étrier et l'extrémité distale d'extrémité de la poche comprennent un voile inextensible reliant ces deux éléments sur toute leur longueur. Ceci rend l'effort de traction homogène sur toute la hauteur de la poche et évite tout désaxement de l'étrier; l'axe de ce dernier doit en effet se déplacer parallèlement à lui-même pour éviter tout glissement du tambour sur la poche.

Les moyens d'entraînement comprennent avantageusement un moteur électrique dont le corps extérieur du stator est solidaire du tambour, et dont le rotor entraîne l'étrier par l'intérmèdiaire d'un réducteur (dans le cas d'un moteur normal) ou sans réducteur (dans le cas d'un moteur couple), ainsi que des moyens pour déterminer la position angulaire relative de l'étrier par rapport au tambour, coopérant avec des moyens pour interrompre l'alimentation des moyens d'entraînement lorsqu'une valeur angulaire prédéterminée est atteinte.

Le moteur électrique est alors de préférence un moteur réversible qui est alimenté en tension inverse de valeur réduite une fois la valeur angulaire prédéterminée atteinte, pour assister le retour des éléments de la pompe à leur position l'origine. Un retour libre, sans assistance, serait certes possible, sous le seul effet de la pression de remplissage (pression veineuse) et de l'élasticité de la poche, mais ce retour serait trop long pour correspondre au rythme cardiaque normal; le temps de retour serait en outre sujet à l'influence de nombreux paramètres difficilement maîtrisables. C'est pourquoi on préfère contrôler le retour à la position d'origine — et donc le temps de remplissage.

Dans ce cas, la tension inverse de valeur réduite peut être une tension variable, de manière à ajuster la durée de la phase de retour en fonction d'une information de consigne de fréquence de fonctionnement, qui peut être par exemple un signal physiologique fourni par le coeur droit (dans le cas d'une assistance ventriculaire gauche), ou encore un signal prélevé au niveau d'une oreillette qui aura été conservée, ou encore une fréquence prédéterminée non physiologique.

Si l'on envisage une prothèse cardiaque totale, la pompe de l'invention peut comprendre deux poches fermées, avec chacune une valve d'entrée et une valve de sortie propres, ces deux poches étant adjacentes à l'intérieur de la coque et ayant leurs extrémités proximales respectives reliées ensemble au tambour, et leurs extrémités distales respectives reliées ensemble à l'étrier par une liaison de traction commune.

Notamment, on peut prévoir que ce soit le ventricule gauche qui soit en contact direct acec le tambour, le ventricule droit n'étant comprimé qu'indirectement, par le ventricule gauche et non par le tambour.

Un tel fonctionnement permet de se rapprocher des conditions de fonctionnement d'un coeur naturel, dans lequel c'est le ventricule gauche qui fournit l'essentiel de l'énergie cardiaque de pompage; il respecte également le synchronisme de fonctionnement entre les deux ventricules.

D'autres caractéristiques et avantages de l'invention apparaîtront à la lecture de la description détaillée ci-dessous, faite en référence aux figures annexées, sur lesquelles:

les figures 1 à 3 sont des vues avec arrachement partiel de la pompe de l'invention, respectivement en perspective, de face et de côté,

la fig. 4 est une vue en coupe schématique, selon la ligne IV-IV de la fig. 2,

les figures 5 à 10 illustrent la cinématique de fonctionnement de l'invention,

la fig. 11 est une vue en coupe, homologue de la fig. 4, pour une assistance ventriculaire double, correspondant à une prothèse totale,

la fig. 12 montre, de façon schématique, un exemple de circuit de commande pour le moteur de la pompe de l'invention.

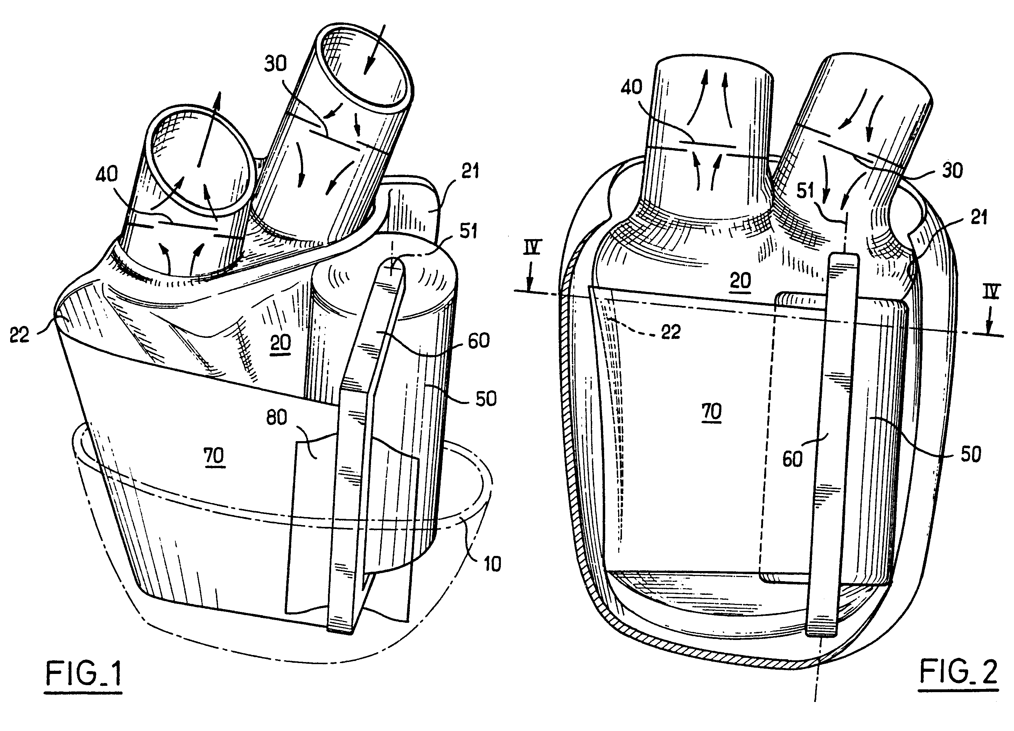

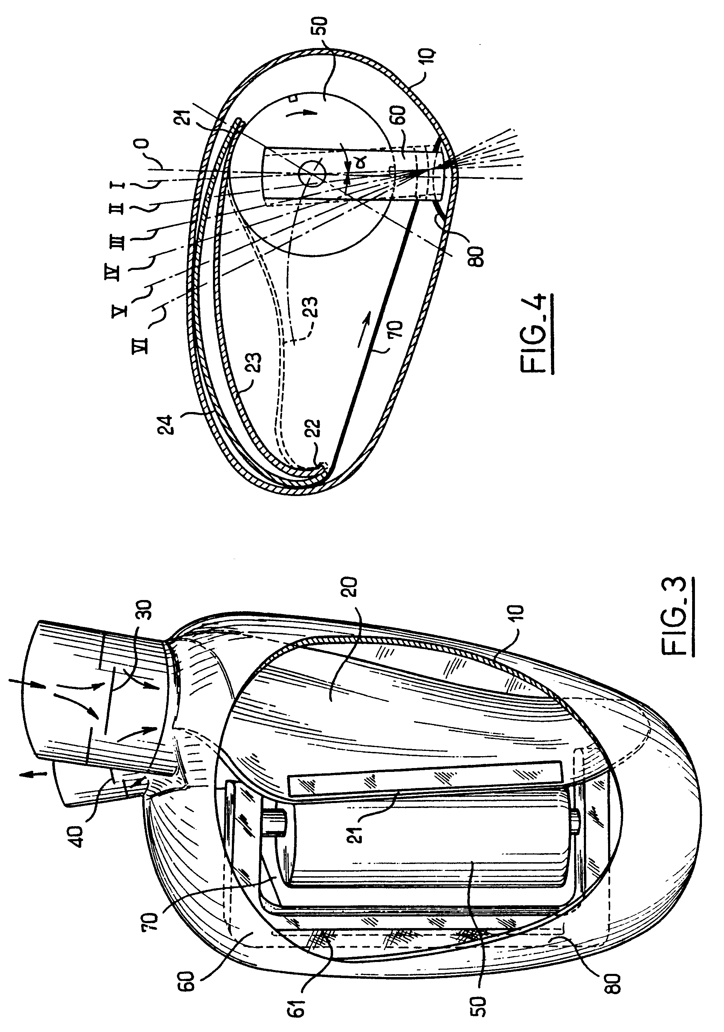

Sur les figures 1 à 4, on peut voir l'ensemble de la pompe, située à l'intérieur d'une coque 10 formant enveloppe extérieure, et partiellement arrachée sur les figures 1 à 3. Une poche 20, de forme libre plate, s'étend entre deux régions d'extrémité 21 et 22; en coupe transversale (fig. 4) elle a une forme générale de croissant, dont le degré d'aplatissement dépend de la pression du liquide de remplissage. En particulier, sur la fig. 4, la ligne en trait continu représente le profil de la poche à vide, tandis que les lignes tiretées représentent la forme de la poche en charge, sous une pression de 50 mm Hg (6600 Pa) par exemple, pour laquelle sa capacité est de l'ordre de 75 cm$^3$.

Cette poche est alimentée en fluide sanguin par une valve d'entrée 30 d'un type classique (disque basculant par exemple); le remplissage se faisant sans aspiration, cette valve d'entrée doit pouvoir s'ouvrir pour une pression différentielle de l'ordre de 10 à 12 mm Hg (1300 à 1600 Pa).

La poche 20, comprimée, refoule le fluide sanguin par l'intermédiaire d'une valve de sortie 40, qui peut être également d'un type classique, mais susceptible de supporter une pression inverse d'au moins 140 mm Hg (19 000 Pa). Cette valeur correspond à la pression artérielle maximale pour un sujet ayant un réseau artériel sain; elle peut être variable selon la résistance présentée par les vaisseaux périphériques, et notamment en cas d'athérosclérose.

L'extrémité proximale 21 de la poche est fixée à un tambour 50, auquel est articulé un étrier 60 autour d'un axe 51. L'autre extrémité, distale, 22 de la poche est reliée à l'étrier 60 par un voile inextensible 70.

En outre, avantageusement, l'étrier 60 est relié à la coque 10 au moyen d'une liaison 80. Cette liaison n'est pas une liaison rigide; elle définit simplement pour l'étrier une zone de débattement par rapport à la coque, en permettant au moins un pivotement de la partie verticale de celui-ci autour d'un axe longitudinal. Cette liaison peut par exemple être réalisée, comme représenté, au moyen d'un voile de tissu synthétique collé ou suturé à la coque 10, et enfilé dans une fente 61 (fig. 3) de la partie centrale de l'étrier; une telle liaison sur une grande partie de la longueur de l'étrier permet d'assurer une bonne homogénéité du couplage coque/étrier.

On rappellera cependant que cette liaison coque-étrier n'est pas un élément nécessaire de la combinaison de moyens proposée par l'invention: cette combinaison vise un montage intrinsèquement «flottant», c'est-à-dire capable de foctionner sans point d'appui extérieur (de la même façon qu'une poche contractile naturelle).

En l'absence de liaison coque-étrier, la coque ne joue ainsi aucun rôle mécanique, et n'est prévue que comme simple organe de protection.

Cette variante peut d'ailleurs être préférée dans certains cas, notamment lorsque l'organe implanté risque d'être soumis à des sollicitations extérieures (compressions thoraciques par exemple) qui pourraient affecter son bon fonctionnement: dans ce cas, on arme la coque (par exemple au moyen d'une armature en forme de treillis métallique) pour renforcer son rôle protecteur; la compression d'une coque trop souple pourrait en effet amener une limitation préjudiciable de la course de fonctionnement du dispositif. Pour éviter des mouvements erratiques du dispositif à l'intérieur de la coque ainsi rigidifiée, on peut par exemple fixer la paroi externe 24 (fig. 4) de la poche à la coque par un point de collage; cette liaison coque-poche n'a cependant aucun rôle fonctionnel de couplage des deux éléments; elle sert seulement à limiter le débattement pendant le fonctionnement.

En ce qui concerne les matériaux utilisés, la coque est faite d'un matériau biocompatible tel qu'un caoutchouc de silicone ou un polyuréthane, qui permettent d'associer à la forme géométriquement déformable de la coque un matériau présentant une mémoire propre. Si l'on ne recherche pas d'élasticité propre, il est possible d'armer le matériau choisi au moyen d'un tissu synthétique copolymérisé.

La poche 20 est également réalisée en un matériau biocompatible et élastiquement déformable, par exemple un caoutchouc de silicone tel que le Silastic®. Ce matériau, en contact avec le sang, est connu pour ses faibles risques d'accidents thromboemboliques, à condition que son état de surface soit extrêmement soigné, ce qui nécessite par exemple un moulage sur des surfaces soigneusement polies. On choisira pour la poche une épaisseur de l'ordre de 1 à 2 mm, qui rendra celle-ci incassable même en présence de points de calcification.

Sur l'exemple représenté, la poche est formée (figures 3 et 4, notamment) de deux feuilles planes superposées et soudées ensemble à leur périphérie, ainsi qu'aux valves 30 et 40. Au moment de leur mise en place dans la coque, les feuilles sont courbées (cette courbure étant encore accentuée par la tension du voile 70), ce qui leur donne la forme représentée fig. 4 en section, correspondant à vide, à une «cavité semi-virtuelle» comme explicité plus haut. Les zones d'extrémité 21 et 22 ne sont pas nécessairement des bords parallèles, ni même rectilignes. Sur l'exemple représenté, les deux feuilles constituant la poche ont une forme sensiblement trapézoïdale, les bords 21 et 22 formant les côtés non parallèles. D'autre formes peuvent cependant être envisagées en fonction de nombreux paramètres tels que la capacité de la poche, la forme du tambour 50, la recherche d'une meilleure logeabilité, ... En outre, la forme de la poche est choisie pour permettre une éjection avec une faible déformation d'ensemble, (comme on le verra à l'examen des figures 4 à 10), sans pliage du matériau, et avec un faible déplacement relatif des valves 30 et 40, qui ne doivent subir qu'un minimum de contraintes de la part de la poche à laquelle elles sont reliées.

Il est également possible d'envisager un moulage monobloc de la poche 20, ce qui évite de prévoir un raccordement le long des zones d'extrémité 21 et 22.

En ce qui concerne le tambour 50, celui-ci a été représenté sous la forme d'un cylindre de révolution tournant autour de son axe. De nombreuses variantes peuvent être cependant envisagées, soit que le cylindre de révolution tourne autour d'un axe excentré par rapport à son axe propre, soit qu'il soit engendré par une génératrice autre qu'une droite, soit encore qu'il s'agisse d'un cylindre qui ne soit pas de révolution.

On peut encore prévoir un tambour constitué d'une fraction de cylindre de révolution (car en fait seule une portion de la surface du cylindre vient en contact avec la poche), ou toute autre forme appropriée.

Cette forme du tambour dépend en fait de sa position par rapport aux valves (la partie supérieure du cylindre doit les déformer peu), du volume et de la forme de la poche (pour éviter des volumes morts), ainsi que de la progressivité de l'effort de compression. De même l'axe 51 de rotation peut être incliné (cf. fig. 2) par rapport à la verticale, pour permettre une meilleure évacuation de la poche vers la valve de sortie.

Dans tous les cas, la fonction essentielle du tambour est de coopérer avec la poche pour permettre l'enroulement — sans glissement relatif des surfaces — autour de la périphérie du tambour. La compression de la poche qui en résultera se fera toujours avec un frottement minimum, les coefficients de frottement de roulement étant toujours très faibles, en l'absence de friction.

La liaison de traction 70 est réalisée par un voile inextensible en tissu synthétique, silicone par exemple. Cette liaison réalisée ainsi sur toute la longeur de la partie centrale de l'étrier permettra à ce dernier de tendre à rapprocher du tambour l'extrémité 22 de la poche, tout en guidant le tambour pour qu'il roule sur la poche, sans glisser dessus.

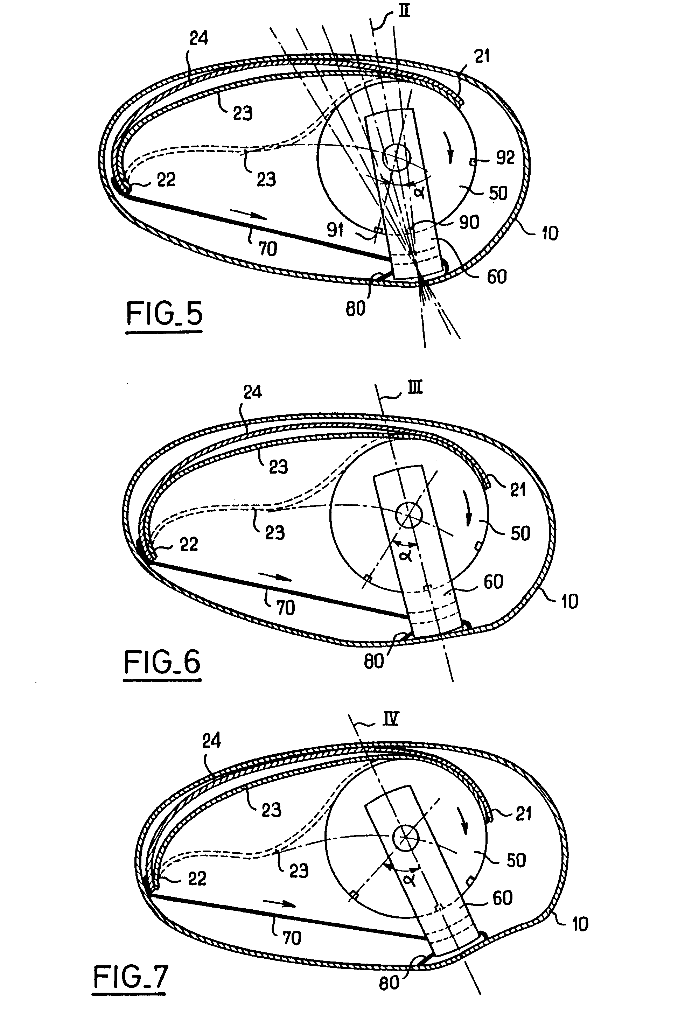

Le moteur utilisé pour entraîner en rotation relative, sur une fraction de tour, l'étrier par rapport au tambour peut être, dans un premier mode de réalisation, un moteur-couple associé à un réducteur (pour disposer d'un couple important en bout d'arbre). Dans ce cas, il est important d'éviter toute dérive angulaire qui amènerait progressivement le tambour en butée contre la coque. Pour recaler l'ensemble après chaque fraction de tour, on peut notamment utiliser un capteur magnétique 90 disposé sur l'étrier (visible fig. 5), qui détecte la présence d'aimants 91, 92 portés par le tambour et correspondant aux positions extrêmes autorisées.

L'angle maximal de rotation est de l'ordre de 100° dans l'exemple représenté, mais cette valeur dépend dans une large mesure de la géométrie de l'ensemble moteur-coque-poche.

Dans un second mode de réalisation, il est possible d'utiliser un moteur sans balais, commandé par un train d'impulsions, ce qui évite l'emploi d'un détecteur de position angulaire, le déplacement relatif du stator et du rotor étant proportionnel au nombre d'impulsions envoyées.

Il est cependant possible, dans ce second mode de réalisation, de prévoir par sécurité un détecteur subsidiaire de position angulaire, qui viendra doubler le système de comptage du nombre d'impulsions.

Une autre sécurité peut être constituée par un détecteur de surintensité, qui coupe l'alimentation du moteur si celui-ci ne parvient pas à atteindre sa position angulaire extrême. Ce système de sécurité permettra quand même de continuer à faire fonctionner le dispositif.

Le moteur lui-même est choisi parmi les modèles miniaturisés à rendement élevé, par exemple utilisant des aimants au samarium-cobalt. Le problème du rendement du moteur prend une acuité particulière dans l'application envisagée, dans la mesure où le fonctionnement est un fonctionnement intermittent et non une rotation continue: Le besoin en énergie est précisément le plus élevé au moment du démarrage, c'est-à-dire quand le moteur est en court-circuit et donc quand son rendement instantané est le plus faible.

L'alimentation du moteur (non représentée) peut être réalisée par des batteries implantées rechargeables, ou des batteries extérieures avec un passage percutané ou, de préférence, transcutané (couplage électromagnétique).

De plus, l'excellent rendement de la pompe de l'invention permet d'envisager l'emploi d'un générateur isotopique implanté. En effet, bien qu'un tel dispositif ait en lui-même un faible rendement, la puissance qu'il délivre reste suffisante pour alimenter la pompe de l'invention, dont la puissance est voisine de la puissance théorique physiologique (quelques watts). Le dégagement de chaleur d'un générateur isotopique de faible puissance reste compatible avec la thermorégulation naturelle.

On va maintenant décrire le fonctionnement dynamique de la pompe de l'invention, en référence aux figures 4 à 10.

Sur la fig. 4, on a représenté un certain nombre de positions désignées 0, I.....VI, les positions II à VI de l'étrier correspondant aux figures 5 à 9.

En position 0, la poche 20 est remplie de fluide sanguin (profil représenté en tiretés sur les figures), et le tambour 50 n'appuie pratiquement pas sur la poche; par ailleurs, le voile 70 est lâche.

Dès la mise en route du moteur, le tambour va venir appuyer contre la paroi 23 de la poche, provoquant par réaction le déplacement de l'étrier de la position 0 à la position I, jusqu'à ce que le voile 70 soit mis en tension. La poche 20 vient alors en butée contre la face intérieure de la coque 10, créant un point d'appui (au moins pendant la phase de démarrage). Par la suite, le tambour et l'étrier vont suivre un mouvement de moindre résistance, réalisant une auto-adaptation de façon à équilibrer la réaction de compression de la poche par le tambour et la réaction de traction par le voile. En effet:

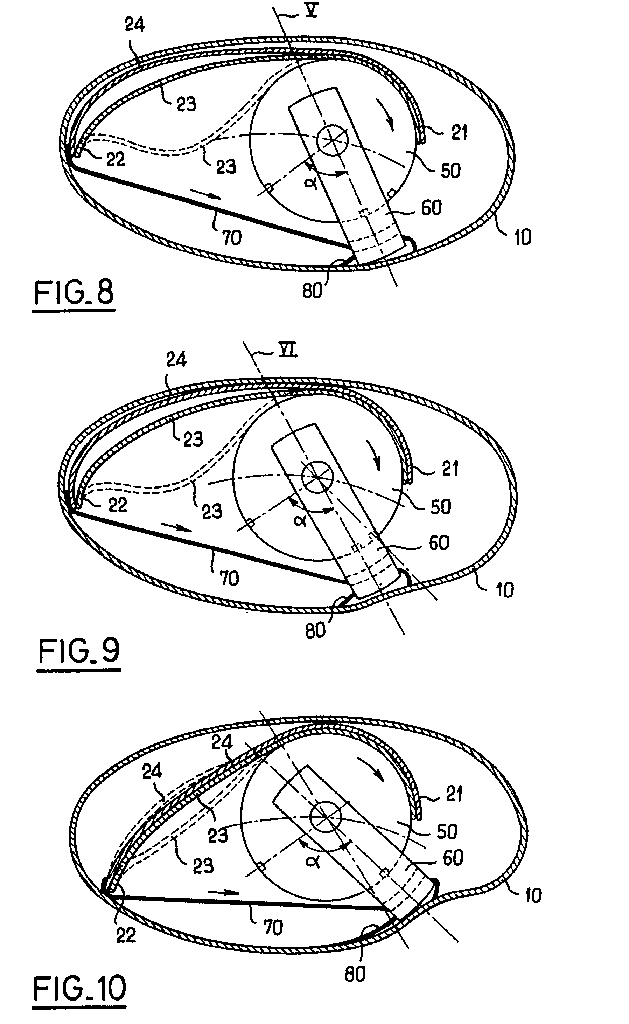

si la poche offre une résistance importante à la compression par le roulement du tambour 50, ce dernier aura tendance à être immobilisé, ce qui provoquera essentiellement un déplacement (déplacement absolu) de l'étrier 60. Ce déplacement de l'étrier va accroître la tension sur le voile 70, jusqu'à ce que cette tension équilibre la réaction de la poche à la compression. Une telle phase où la résistance à la compression est élevée se produit par exemple au tout début de l'éjection (positions 0 et I de la fig. 4, où le voile est faiblement tendu), ou encore en fin d'éjection (figures 9 et 10), ou encore dans le cas d'une pression sanguine élevée,

si par contre la résistance de compression opposée par la poche est relativement faible, c'est le tambour 50 qui aura tendance à rouler sur la poche pour la déformer et l'enrouler à sa périphérie; l'étrier fait alors office de simple support d'axe. Une telle situation est par exemple celle correspondant aux figures 5 à 8 (où l'on voit que la longueur de poche enroulée sur le tambour augmente rapidement), ou encore dans le cas d'une faible pression sanguine.

Le montage «flottant» du système permet d'obtenir une inversion permanente, autour d'une position d'équilibre, entre le point d'appui et le point d'application de la force exercée par l'ensemble tambour-étrier qui forme un levier articulé: tantôt le tambour, en appui sur la poche, sert d'appui à l'étrier pour qu'il exerce sa traction sur l'extrémité distale de la poche et rabatte cette dernière vers le tambour; tantôt l'étrier, maintenu en place par la traction exercée sur le voile 70, sert de support au tambour qui tourne en enroulant la poche à sa périphérie.

De la même façon, en cas de venue en contact de la poche avec la coque, un frottement à cet endroit tent à immobiliser la poche. Par réaction, le tambour se met à rouler sur la poche, et l'enroulement résultant va rabattre cette dernière vers l'intérieur, allégeant d'autant le contact avec la coque et le frottement à cet endroit.

Dans tous les cas, l'ensemble «fuit le frottement», c'est-à-dire qu'il se déforme jusqu'à ce que le tambour soit dans la position qui lui assure le frottement minimal, qui est essentiellement un frottement de roulement de valeur très faible.

Ce système auto-adaptif explique les excellentes performances d'ensemble du dispositif, notamment son rendement très élevé de transformation d'énergie électrique en énergie pneumatique de pompage.

On peut par ailleurs noter que le déplacement absolu du tambour est très faible, car c'est principalement la poche qu'on enroule sur le tambour (soit par enroulement direct, soit en ramenant l'extrémité distale vers le tambour), à la différence des pompes péristaltiques classiques utilisées dans les circulations extra-corporelles, ou c'est la cavité déformable qui est immobile.

La durée totale de la phase d'éjection (systole) est de l'ordre de 350 ms — correspondant à la séquence des figures 4 à 10 —; en fin déjection, il est avantageux de laisser subsister un voile sanguin de 1 à 2 mm (fig. 10) entre les deux parois de la poche, pour éviter tout risque d'hémolyse.

La phase de remplissage (diastole) se fait par retour de l'ensemble à la position initiale. Comme on l'a indiqué plus haut, ce retour résulte de la pression veineuse, de l'élasticité propre de la poche, de l'énergie restituée après déformation de la coque, ainsi que d'une tension inverse appliquée au moteur, pour assister cette phase de retour.

Avantageusement, la durée de la phase de retour peut être modifiée, en conservant une durée constante pour la phase d'éjection. De cette manière, la durée totale sera une durée variable, qui pourra par exemple être asservie à un signal de consigne de rythme cardiaque.

L'énergie fournie par le moteur au cours de la phase de retour est très faible, de l'ordre de 1/100ème de celle nécessaire au cours de la phase d'éjection; il s'agit simplement d'accompagner le mouvement de la poche qui se déroule du tambour sous l'effet du remplissage naturel par la pression veineuse. On peut remarquer que la distribution d'énergie dans le temps est comparable au fonctionnement physiologique d'un coeur naturel, pour lequel l'énergie développée est pratiquement nulle pendant les 2/3 de la période cardiaque.

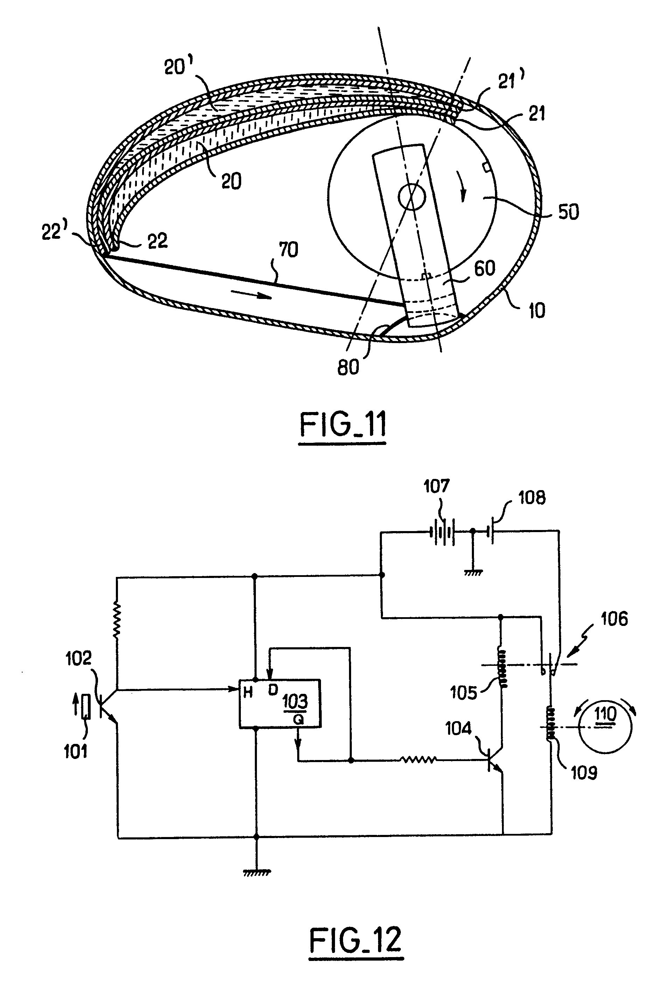

La figure 11 montre un mode de réalisation dans lequel il est prévu non plus une, mais deux poches 20, 20' superposées, avec chacune leurs valves propres (non représentées) et qui sont susceptibles de constituer les deux ventricules d'une prothèse totale implantable.

Ces deux poches sont superposées à l'intérieur d'une même coque 10; leurs deux extrémités proximales 21, 21' sont réunies ensemble et au tambour 50, tandis que leurs extrémités distales 22, 22' sont réunies ensemble et reliées à l'étrier à 60 par un voile unique 70. (Les parois internes, adjacentes, des poches 20, 20' peuvent bien entendu être confondues en une paroi séparatrice unique).

De la sorte, le mouvement du tambour et de l'étrier va produire une compression simultanée et sensiblement synchrone des deux poches 20, 20': la poche 20 (celle placée le plus à l'intérieur) est comprimée directement du fait du contact avec le tambour; l'autre poche 20' n'est par contre comprimée qu'indirectement en raison de l'interposition de la première poche 20 entre sa paroi tournée vers l'intérieur et la surface du tambour. L'effort de compression sera donc moindre pour cette seconde poche, ce qui résultera en une plus faible puissance de pompage et une plus faible pression d'éjection.

On peut tirer parti de cette particularité en prévoyant que la poche 20 constituera le ventricule gauche de la prothèse, tandis que la poche 20' constituera le droit. La différence des puissances et des pressions de pompage entre les deux poches correspondra alors à la réalité physiologique: en effet, le ventricule droit, qui n'assure que la circulation pulmonaire, fournit un effort très inférieur à celui du ventricule gauche, qui a en charge l'ensemble de la circulation périphérique.

La fig. 12 représente schématiquement un circuit électronique permettant la commande du fonctionnement de la pompe précédemment décrite, qu'il s'agisse d'une pompe mono- ou bi-ventriculaire.

Un aimant permanent 101 (alternativement l'un ou l'autre des deux aimants 91 et 92 représentés fig. 5) passe devant un détecteur 102, par exemple un détecteur à effet Hall, chaque fois qu'une des positions extrêmes est atteinte.

Pour inverser le sens de rotation du moteur à ce moment-là, il est prévu une bascule D 103 dont l'entrée d'horloge H est excitée par la variation de tension aux bornes du détecteur 102. La sortie Q (également reliée à l'entrée D) de la bascule pilote un transistor 104 de commande de l'enroulement 105 d'un relais inverseur. Les contacts 106 de ce relais commutent alternativement l'enroulement 109 du moteur 110 à deux surces de tension 107, 108 de polarités opposées (la source 108, correspondant à la phase de retour, est avantageusement de tension plus faible que la source 107, comme indiqué plus haut).

De cette manière, le basculement du circuit 103, et du relais — entraînant l'inversion de sens du monteur — aura lieu chaque fois qu'une position extrême sera atteinte, détectée par le passage de d'un ou de l'autre aimant au droit du détecteur 102.

Bien entendu, le relais électromécanique à enroulement et contacts représenté peut être remplacé par un relais purement statique à jonction semiconductrice.

*Résultats Expérimentaux*

L'expérimentation in vivo d'une pompe d'assistance gauche telle qu'ici décrite (dans la variante avec coque renforcée et absence de couplage coque-étrier) a été réalisée sur le porc: ce modèle animal dont les constantes physiologiques cardiovasculaires et respiratoires sont proches de celles de l'homme peut de plus être choisi avec un poids très similaire.

Sur le plan chirurgical, la greffe est réalisée sans utilisation de circulation extra corporelle par clampage latéral de l'aorte et de l'oreillette gauche. Cette facilité opératoire qui ne sera pas utilisée chez l'homme rend beaucoup plus délicates les opérations de purge et d'amorçage de la prothèse.

Malgré cette difficulté, le déroulement opératoire a été sans incident sur cinq animaux greffés.

Sur chaque expérience on a essayé successivement soit le clampage de l'aorte descendante, le ventricule artificiel alimentant alors toute la partie inférieure de l'animal, soit le clampage aortique total, le ventricule gauche assurant la totalité de la fonction cardiaque gauche à l'exception des coronaires qui restaient alimentées par le coeur naturel.

Pendant ces opérations de clampage qui permettaient au ventricule gauche de passer d'une fonction d'assistance à une fonction de remplacement, on a pu constater l'adaptation automatique et immédiat de la prothèse aux conditions différentes de pression et de débit.

Ce résultat unique comparé à d'autres prothèses est essentiellement dû à l'absence d'aspiration du ventricule artificiel. Par ailleurs, le taux de remplissage variable du ventricule gauche qui dépend uniquement du retour veineux au niveau de l'oreillette gauche, ne modifie pas les pressions d'éjection qui oscillent suivant le besoin entre 80 et 140 mm de mercure (11 000 et 19 000 Pa); ce taux de remplissage variable confirme le fonctionnement non-aspirant et donc non-volumétrique de la pompe, qui évite précisément tout risque de collapsus.

Après 10 heures d'implatation les analyses effectuées régulièrement ne montrent aucune destruction des globules rouges (hémolyse) ni aucune aggrégation plaquettaire. Ce résultat est également fondamental.

Toutes les analyses biologiques restent normales.

Parallèlement, on a effectué la greffe de prothèses inertes (fantôme) pour vérifier la compatibilité des matériaux constitutifs avec l'organisme. A ce jour, après un mois d'implantation, aucune réaction anormale n'est observée.

Les courbes tension et courant du ventricule gauche en fonctionnement, obtenues par un traceur automatique, permettent le calcul de la consommation moyenne de la pompe qui est de 2,25 watts pour un débit de 50 cm$^3$ par systole et une pression de 140 mm de mercure.

**Revendications**

1. Une pompe sanguine, notamment implantable, comprenant:

une coque extérieure (10),

au moins une poche fermée (20) élastiquement déformable, alimentée en fluide sanguin par une valve d'entrée (30), et susceptible de refouler ce fluide par une valve de sortie (40),

ladite pompe étant caractérisée en ce que ladite poche a une forme libre sensiblement plate et est placée contre la paroi intérieure de la coque de façon à présenter deux parois (23, 24) qui, ainsi courbées, tournent toutes deux leur concavité vers l'intérieur de la coque, ces deux parois s'étendant entre deux régions allongées longitudinales, formant extrémités proximale (21) et distale (22),

et en ce qu'elle comprend en outre: un tambour (50), à la périphérie duquel est reliée l'extrémité proximale (21) de la poche,

un étrier (60), monté à rotation sur l'axe longitudinal (51) du tambour,

des moyens (70) pour réaliser une liaison de traction entre l'étrier (60) et l'extrémité distale (22) de la poche,

des moyens pour entraîner en rotation relative et antagoniste, sur une fraction de tour, l'étrier (60) et le tambour (50), dans un sens permettant à ce dernier de venir en contact de roulement avec la paroi (23) de la poche, tandis que l'étrier maintient l'extrémité distale et tend à la rapprocher du tambour,

de manière à permettre la compression de la poche sous l'effet d'un enroulement de celle-ci sur le tambour,

et de manière que les positions successives de l'ensemble tambour-étrier suivent un mouvement de moindre résistance entre la réaction de compression de la poche et la réaction de traction de l'extrémité distale de la poche.

2. Une pompe sanguine selon la revendication 1, caractérisée en ce qu'il est prévu en outre une liaison (80) entre l'étrier (60) et la coque extérieure (10), cette liaison étant une liaison lâche permettant un pivotement de l'étrier par rapport à la coque, au moins autour d'un axe longitudinal.

3. Une pompe sanguine selon l'une des revendications 1 et 2, caractérisée en ce que la coque extérieure est géométriquement déformable dans son ensemble.

4. Une pompe sanguine selon la revendication 3, caractérisée en ce que le matériau constituant la coque (10) est un matériau déformable possédant une mémoire propre.

5. Une pompe sanguine selon l'une des revendications 2 à 4, caractérisée en ce que les moyens (70) pour réaliser une liaison de traction entre l'étrier et l'extrémité distale de la poche comprennent un voile inextensible reliant ces deux éléments sur toute leur longueur.

6. Une pompe sanguine selon l'une des revendications 1 à 5, caractérisée en ce que les moyens d'entraînement comprennent un moteur électrique dont le corps extrérieur du stator est solidaire du tambour, et dont le rotor entraîne l'étrier.

7. Une pompe sanguine selon la revendication 6, caractérisée en ce qu'elle comprend en outre des moyens pour déterminer la position angulaire relative de l'étrier par rapport au tambour, coopérant avec des moyens pour interrompre l'alimentation des moyens d'entraînement lorsqu'une valeur angulaire prédéterminée est atteinte.

8. Une pompe sanguine selon la revendication 7, caractérisée en ce que le moteur électrique est un moteur réversible qui est alimenté en tension inverse de valeur réduite une fois la valeur angulaire prédéterminée atteinte, pour assister le retour des éléments de la pompe à leur position d'origine.

9. Une pompe sanguine selon la revendication 8, caractérisée en ce que la tension inverse de valeur réduite est une tension variable, de manière à ajuster la durée de la phase de retour en fonction d'une information de consigne de fréquence de fonctionnement.

10. Une pompe sanguine selon l'une des revendications précédentes, caractérisée en ce qu'elle comprend deux poches fermées (20, 20'), avec chacune une valve d'entrée et une valve de sortie propres, ces deux poches étant adjacentes à l'intérieur de la coque et ayant leurs extrémités proximales respectives reliées ensemble au tambour, et leurs extrémités distales respectives reliées ensemble à l'étrier par une liaison de traction commune, la poche (20) directement comprimée par le contact du tambour formant le ventricule gauche, l'autre poche (20'), indirectement comprimée par suite de l'interposition de la première poche, formant le ventricule droit.

**Patentansprüche**

1. Implantierbare Blutpumpe, die folgendes enthält:

eine äussere Schale (10),

mindestens eine elastisch verformbare geschlossene Tasche (20), die über ein Einlassventil (30) mit Blutflüssigkeit versorgt wird und dieses Fluid über ein Auslassventil (40) wieder abgibt,

dadurch gekennzeichnet, dass diese Tasche eine weitgehend flache freie Form hat und an der Innenwand der Schale so angeordnet ist, dass sie zwei Wände (23, 24) aufweist, die so gekrümmt ihre konkave Seite zur Aussenseite der Schale drehen, wobei sich diese beiden Wände zwischen zwei Längsbereichen erstrecken, welche ein proximales Ende (21) und eine distales Ende (22) bilden, und dass sie weiterhin folgendes aufweist:

eine Trommel (50), an deren Peripherie das proximale Ende (21) der Tasche befestigt ist,

einen Bügel (60), der drehend auf der Längsachse (51) der Trommel montiert ist,

Mittel (70), um eine Zugverbindung zwischen dem Bügel (60) und dem distalen Ende (22) der Tasche herzustellen

Mittel, um den Bügel (60) und die Trommel (50) über einen Bruchteil einer Umdrehung in relative und antagonistische Drehebewegung in einer Richtung versetzen, die der Trommel erlaubt, mit der Wand (23) der Tasche in rollenden Kontakt zu treten, während der Bügel das distale Ende festhält und versucht, es an die Trommel anzunähern, um eine Kompression der Tasche unter der Wirkung des Aufrollens auf die Trommel zu ermöglichen, und zwar in einer Weise, dass die jeweiligen Positionen des Zusammenbaus aus Trommel und Bügel einer Bewegung des geringsten Widerstandes zwischen der Kompressionsreaktion der Tasche und der Zugreaktion des distalen Endes des Tasche folgen.

2. Blutpumpe nach Anspruch 1, dadurch gekennzeichnet, dass ausserdem eine Verbindung (80) zwischen dem Bügel (60) und der äusseren Schale (10) vorgesehen ist, wobei diese Verbindung eine lasche Verbindung ist, die ein Schwenken des Bügels im Vergleich zur Schale mindestens um eine Längsachse erlaubt.

3. Blutpumpe nach einem der Ansprüche 1 oder 2, dadurch gekennzeichnet, dass die äussere Schale insgesamt geometrisch verformbar ist.

4. Blutpumpe nach Anspruch 3, dadurch gekennzeichnet, dass der Werkstoff, aus dem die Schale (10) geformt ist, ein verformbarer Werkstoff mit eigenem Gedächtnis ist.

5. Blutpumpe nach einem der Ansprüche 1 bis 4, dadurch gekennzeichnet, dass die Mittel (70) für die Herstellung einer Zugverbindung zwischen dem Bügel und dem distalen Ende der Tasche einen nicht dehnbaren Schleier aufweisen, welcher diese beiden Elemente über ihre gesamte Länge verbindet.

6. Blutpumpe nach einem der Anprüche 1 bis 5, dadurch gekennzeichnet, dass die Antriebsmittel einen Elektromotor aufweisen, dessen äusseres Statorgehäuse mit der Trommel fest verbunden ist und dessen Rotor den Bügel antreibt.

7. Blutpumpe nach Anspruch 6, dadurch gekennzeichnet, dass sie ausserdem Mittel aufweisst, um die relative Winkelposition des Bügels im Vergleich zur Trommel zu bestimmen, um die Speisung der Antriebsmittel zu unterbrechen, wenn ein vorbestimmter Winkelwert erreicht ist.

8. Blutpumpe nach Anspruch 7, dadurch gekennzeichnet, dass der Elektromotor ein Umkehrmotor ist, der mit umgekehrter Spannung geringen Wertes gespeist wird, wenn der vorbestimmte Winkelwert erreicht ist, um die Rückkehr der Bestandteile der Pumpe in ihre Ausgangsstellung zu unterstützen.

9. Blutpumpe nach Anspruch 8, dadurch gekennzeichnet, dass die umgekehrte Spannung geringen Wertes eine variable Spannung ist, um die Dauer der Rückkehrphase in Abhängigkeit von einem Informationssignal für die Funktionsfrequenz zu steuern.

10. Blutpumpe nach einem der vorausgegangenen Ansprüche, dadurch gekennzeichnet, dass sie zwei geschlossene Taschen (20, 20') aufweist, die jeweils ein eigenes Einlass- und Auslassventil enthalten, wobei diese beiden Taschen nebeneinander in der Schale angeordnet sind und ihre beiden proximalen Enden zusammen mit der Trommel verbunden sind, während ihre beiden distalen Enden jeweils gemeinsam mit dem Bügel über eine gemeinsame Zugverbindung verbunden sind, wobei die direkt durch Berührung mit der Trommel komprimierte Tasche (20) das linke Ventrikel bildet, während die andere Tasche (20'), die indirekt durch die Zwischenlagerung der ersten Tasche komprimiert wird, das rechte Ventrikel bildet.

**Claims**

1. A blood pump, particularly of implantable type, comprising:

an outer body (10),

at least one elastically deformable sealed pocket (20) supplied with blood fluid by an inlet valve (30) and able to force said fluid back through an outlet valve (40),

said pump being characterised in that said pocket has a substantially flat free shape and is placed against the inner wall of the body so as to present two walls (23, 24) which, thus curved, have concave surfaces facing the inside of the body, said two walls extending between two longitudinally elongate regions, defining proximal (21) and distal (22) edges,

and in that the pump comprises in addition:

a drum (50), to the periphery of which the proximal edge (21) of the pocket is connected,

a clamp (60) mounted so as to be rotatable about the longitudinal axis (51) of the drum,

means (70) for providing a traction connection between the clamp (60) and the distal edge (22) of the pocket,

means for driving the clamp (60) and the drum in relative rotation in opposite senses through a fraction of a revolution in a sense enabling the drum to come into rolling contact with the wall (23) of the pocket, while the clamp holds the distal edge and tends to bring it nearer the drum,

such as to permit the compression of the pocket under the rolling action of the pocket on the drum,

and such that the successive positions of the drum clamp unit follow a movement of lesser resistance between the pocket compression reaction and the traction reaction of the distal edge of the pocket.

2. A blood pump according to claim 1, characterised in that a connection (80) is additionally provided between the clamp (60) and the outer body (10), said connection being a loose connection allowing pivoting of the clamp relative to the body, at least about a longitudinal axis.

3. A blood pump according to one of claims 1 and 2, characterised in that the outer body is geometrically deformable as a unit.

4. A blood pump according to claim 3, characterised in that the material of the body (10) is a deformable material with its own memory.

5. A blood pump according to one of claims 1 to 4, characterised in that the means (70) for providing a traction connection between the clamp and the distal edge of the pocket comprises an inextensible sheet connecting these two elements throughout their length.

6. A blood pump according to one of claims 1 to 5, characterised in that the driving means comprise an electric motor the outer body of the stator of which is solid with the drum, and the rotor of which drives the clamp.

7. A blood pump according to claim 6, characterised in that it comprises in addition means for determining the relative angular position of the clamp with respect to the drum, co-operating with means for interrupting the supply to the driving means when a predetermined angular value is reached.

8. A blood pump according to claim 7, characterised in that the electric motor is a reversible motor supplied with a reverse voltage of reduced magnitude when the predetermined angular value has been reached, in order to assist the return of the pump elements to their original position.

9. A blood pump according to claim 8, characterised in that the reverse voltage of reduced value is a variable voltage, such as to set the duration of the return phase as a fonction of information relating to frequency of operation.

10. A blood pump according to one of the preceding claims, characterised in that it comprises two sealed pockets (20, 20'), each having its own inlet valve and outlet valve, said two pockets being adjacent inside the body and having their respective proximal edges connected together to the drum, and their respective distal edges connected together to the clamp by a common traction connection, the pocket (20) directly compressed by the drum contact defining the left ventricle, the other pocket (20'), indirectly compressed as a result of the interposition of the first pocket, defining the right ventricle.

FIG_1

FIG_2

0 148 661

FIG.3

FIG.4

FIG_5

FIG_6

FIG_7

FIG.8

FIG.9

FIG.10

FIG_11

FIG.12